# EUROPÄISCHE PATENTSCHRIFT

(11) **EP 3 824 950 B1**
(45) Veröffentlichungstag und Bekanntmachung des Hinweises auf die Patenterteilung: **20.04.2022**
(21) Anmeldenummer: 20212121.6
(22) Anmeldetag: 17.01.2019
(51) Int. Cl.: A61N 1/378, A61N 1/362

(54) **ELEKTRONISCHER SCHRITTMACHER**
ELECTRONIC PACEMAKER
STIMULATEUR CARDIAQUE ÉLECTRONIQUE

(30) Priorität: 17.01.2018 DE 102018100998; 18.04.2018 DE 102018205940
(43) Veröffentlichungstag der Anmeldung: 26.05.2021
(62) Teilanmeldung aus: 19152333.1
(73) Patentinhaber: Mehnert, Walter, 85521 Ottobrunn (DE); Theil, Thomas, 82340 Feldafing (DE)
(72) Erfinder: Mehnert, Walter, 85521 Ottobrunn (DE); Theil, Thomas, 82340 Feldafing (DE)
(74) Vertreter: Kilian Kilian & Partner

(56) Entgegenhaltungen:
- WO-A1-2012/013212
- DE-A1- 10 127 807
- US-A- 3 820 090
- MATSUSHITA A ET AL: "Power generating device using compound magnetic wire", JOURNAL OF APPLIED PHYSICS, AMERICAN INSTITUTE OF PHYSICS, US, Bd. 87, Nr. 9, 1. Mai 2000 (2000-05-01), Seiten 6307-6309, XP012050482, ISSN: 0021-8979, DOI: 10.1063/1.372688
- STEFANO SAGGINI ET AL: "Low-Power Energy Harvesting Solutions for Wiegand Transducers", IEEE JOURNAL OF EMERGING AND SELECTED TOPICS IN POWER ELECTRONICS, Bd. 3, Nr. 3, 1. September 2015 (2015-09-01), Seiten 766-779, XP055594705, Piscataway, NJ, USA ISSN: 2168-6777, DOI: 10.1109/JESTPE.2015.2424598

## Beschreibung

Die Erfindung betrifft einen elektronischen Schrittmacher zur Implantation in einen Körper eines Lebewesens.

Schrittmacher, die bestimmungsgemäß vollständig in den menschlichen Körper implantiert werden, sind im Stand der Technik allgemein bekannt und werden bei einer Vielzahl von verschiedenen medizinischen Indikationen eingesetzt.

Beispielsweise werden Schrittmacher bei Erkrankungen des menschlichen Herzen als sogenannte Herzschrittmacher eingesetzt. Die wesentlichen Komponenten eines Herzschrittmachers bilden Elektroden, die mit dem erkrankten Herz des Patienten verbunden werden, und eine Elektronik, die bei Feststellen von Fehlfunktionen des Herzen über die Elektroden Stimulationsimpulse an das Herz abgibt. Permanente Herzschrittmacher werden heute vollständig in den menschlichen Körper implantiert.

Andere Schrittmacher sind beispielsweise Blasenschrittmacher oder Hirnschrittmacher, die ebenfalls Elektroden besitzen, über die eine entsprechende Elektronik Stimulationsimpulse an das Gehirn oder Nervenbahnen abgibt.

Allen Arten von Schrittmachern ist gemeinsam, dass sie eine Batterie zur Versorgung der Elektronik mit elektrischer Energie aufweisen. Diese Batterien sind aber die Schwachstelle bei den bekannten Schrittmachern, weil aufgrund des eingeschränkten Bauraumes der zur Verfügung stehende Energieinhalt der Batterien deren Lebensdauer begrenzt. Gerade solche Schrittmacher sollten ein Leben lang funktionstüchtig sein. Selbstentladung und hoher Strombedarf führen notwendigerweise zu einem Austausch der Batterien. Der Austausch der Batterie ist allerdings mit einem operativen Eingriff verbunden und daher als kritisch zu bewerten.

Ein Einkammer-Herzschrittmacher der neuesten Generation, der über eine Elektrode eine der Herzkammern stimuliert, kann aufgrund seiner Größe direkt in die entsprechende Herzkammer geschoben und dort über seine mehrarmige Elektrode direkt im Herzmuskel verankert werden. Wenn bei diesem Einkammer-Herzschrittmacher die Batterie leer ist, wird ein weiterer in das Herz eingeführt, der die notwendige Stimulation übernimmt. Der erste Einkammer-Herzschrittmacher bleibt Verwachsen im Herz und wird bzw. kann nicht wieder entfernt werden.

Die Batterie des Schrittmachers kontaktlos durch Induktion wieder aufzuladen gestaltet sich problematisch bzw. ist kaum möglich, weil die Eindringtiefe von elektromagnetischen Feldern mit einer für das Aufladen notwendigen Leistungsübertragung bei hohen Frequenzen zu gering ist, vor allem dann, wenn diese ein aus zum Beispiel EMV-Gründen notwendiges Metallgehäuse durchdringen müssten.

Weiterer Stand der Technik findet sich in WO 2012/013212 und "Stefano Saggini et. al.: Low Power Energy Harvesting Solutions for Wiegand Transducers, IEEE JOURNAL OF EMERGING AND SELECTED TOPICS IN POWER ELECTRONICS, Bd. 3, Nr. 3, 1. September 2015, Seiten 766-779, XP055594705, Piscataway, NJ, USA, ISSN: 2168-6777, DOI: 10.1109/JESTPE.2015.2424598".

Vor obigem Hintergrund ist es Aufgabe der vorliegenden Erfindung, einen elektronischen Schrittmacher mit einer verbesserten Energieversorgung zu schaffen.

Diese Aufgabe wird mit einem elektronischen Schrittmacher gemäß Patentanspruch 1 gelöst. Bevorzugte Ausführungsformen sind Gegenstand der abhängigen Patentansprüche.

*Ein elektronischer Schrittmacher gemäß einem Aspekt der Erfindung ist bestimmungsgemäß zur Implantation in einen Körper eines Lebewesens und zur Steuerung einer Körperfunktion vorgesehen.*

Der erfindungsgemäße elektronische Schrittmacher kann beispielsweise ein Herzschrittmacher (Einkammer-Herzschrittmacher, Zweikammer-Herzschrittmacher, Dreikammer-Herzschrittmacher), ein Gehirnschrittmacher, oder Darmschrittmacher sein. Der erfindungsgemäße elektronische Schrittmacher wird vollständig in den Körper des Lebewesens, insbesondere in einen menschlichen Körper, implantiert.

*Der elektronische Schrittmacher gemäß der Erfindung weist einen Elektrodenabschnitt, der bestimmungsgemäß an einem Körperabschnitt zu befestigen ist, auf.* Der Elektrodenabschnitt beinhaltet in Abhängigkeit davon, welchen Zweck der elektronische Schrittmacher übernimmt bzw. welche Körperfunktion er stimulieren soll, eine bestimmte Anzahl von Elektroden. Die Elektroden werden bestimmungsgemäß mit dem Körperabschnitt, beispielsweise Herz oder Gehirn, der zu stimulieren ist, verbunden.

Handelt es sich beispielsweise bei dem erfindungsgemäßen elektronischen Schrittmacher um einen Einkammer-Herzschrittmacher, beinhaltet dieser eine einzige Elektrode, die mit der zu stimulierenden Herzhälfte - beispielsweise Vorhof oder Herzkammer - verbunden wird. Handelt es sich hingegen um einen Zweikammer-Herzschrittmacher, beinhaltet der entsprechende Elektrodenabschnitt zwei Elektroden, die mit der entsprechenden zu stimulierenden Herzhälfte - beispielsweise Vorhof und Herzkammer - verbunden werden. Die Elektroden beinhalten beispielsweise an ihren Enden spiralförmige Abschnitte, die in den Herzmuskel gedreht und so verankert werden.

Allgemein kann es sich bei dem erfindungsgemäßen elektronischen Schrittmacher um einen Herzschrittmacher gemäß einem beliebigen NBG-Code handeln.

Allgemein können die genannten Elektroden beispielsweise Kabelelektroden sein. Insbesondere beinhaltet der Schrittmacher in diesem Zusammenhang bevorzugt pro Kabelelektrode ein Kabel bestimmter Länge, das bestimmungsgemäß zu einem gewünschten Bereich des Körperabschnitts innerhalb des Körpers verlegt werden kann. An dem Ende des Kabels ist der bevorzugt spiralförmige Abschnitt zur Verankerung der Kabelelektrode in dem Bereich des Körperabschnittes ausgebildet.

Alternativ kann der Schrittmacher bzw. der Elektrodenabschnitt auch ohne Kabelelektrode(n) auskommen. In diesem Fall sind die genannten Elektroden auf einer Außenoberfläche des Schrittmacher ausgebildet, wobei der Schrittmacher so implantiert wird, das die Elektroden jeweils an einem Bereich des Körperabschnitts anliegen und/oder dort verankert werden können. Beinhaltet der Schrittmacher eine Vielzahl von auf der Außenoberfläche gebildeten Elektroden, hat der Schrittmacher entsprechende Abmessungen, sodass die auf der Außenoberfläche gebildeten Elektroden an dem jeweiligen Bereich des Körperabschnitts zur Anlage gebracht und/oder dort verankert werden können.

Weiterhin alternativ kann der Elektrodenabschnitt aus einer Kombination aus mindestens einer einzigen Kabelelektrode und aus mindestens einer einzigen auf der Außenoberfläche ausgebildeten Elektrode aufgebaut sein. In diesem Fall wird der Schrittmacher bevorzugt an dem Körperabschnitt angeordnet, sodass die auf der Außenoberfläche gebildete Elektrode mit dem entsprechenden Bereich des Körperabschnitts in Kontakt kommt und/oder dort verankert ist. Die andere Elektrode, d.h. die Kabelelektrode, wird zu einem anderen Bereich des Körperabschnittes geführt und dort verankert bzw. befestigt.

Allgemein ausgedrückt beinhaltet der Elektrodenabschnitt des erfindungsgemäßen elektronischen Schrittmachers eine für die medizinische Indikation notwendige Anzahl von Elektroden.

*Darüber hinaus beinhaltet der erfindungsgemäße elektronische Schrittmacher eine mit dem Elektrodenabschnitt verbundene Elektronik, die eingerichtet ist, einen Impuls (Stimulationsimpuls bzw. Spannungsimpuls) gemäß erfasster Körperdaten zu erzeugen und diesen über den Elektrodenabschnitt an den Körperabschnitt zur Steuerung der Körperfunktion abzugeben.*

Die Elektronik des erfindungsgemäßen elektronischen Schrittmachers ist bevorzugt so ausgestaltet, dass sie die zu stimulierende bzw. zu steuernde Körperfunktion zur Erfassung der Körperdaten überwachen und bei Bedarf den Impuls (Stimulationsimpuls bzw. Spannungsimpuls) über den Elektrodenabschnitt an den Körperabschnitt abgeben kann.

In Abhängigkeit von dem Zweck und der Art des erfindungsgemäßen elektronischen Schrittmachers kann die Elektronik den Impuls kontinuierlich oder bedarfsweise bei Feststellen einer Fehlfunktion des Körpers erzeugen und über den Elektrodenabschnitt ausgeben.

Wenn es sich bei dem erfindungsgemäßen elektronischen Schrittmacher um einen Herzschrittmacher handelt, kann die Elektronik bevorzugt so ausgestaltet sein, dass sie eine oder mehrere Funktionen (Betriebsarten und/oder Frequenzadaption) gemäß NBG-Schrittmachercode aufweist.

*Weiterhin beinhaltet der erfindungsgemäße elektronische Schrittmacher einen Energiespeicher, z.B. einen Akkumulator oder einen Kondensator (beispielsweise ein Gold-Cap), zur Versorgung der Elektronik mit elektrischer Energie, der nach Entladung mit elektrischer Energie wieder aufgeladen werden kann.*

Die Art des Energiespeichers des erfindungsgemäßen elektronischen Schrittmachers kann beliebig gewählt werden. Beispielsweise kann der Energiespeicher ein Akkumulator, vorzugsweise ein Lithium-Ionen-Akkumulator, sein. Alternativ kann der Energiespeicher ein Kondensator mit bevorzugt geringer Selbstentladung sein. Der Energiespeicher kann bevorzugt hermetisch eingekapselt sein, sodass von ihm keine Gefahr für den Körper des Lebewesens ausgeht.

*Erfindungsgemäß beinhaltet der elektronische Schrittmacher einen mit dem Energiespeicher elektrisch verbundenen Ladeimpulserzeugungsabschnitt, der derart eingerichtet ist, dass er an den Energiespeicher einen Ladeimpuls zum Wiederaufladen des Energiespeichers abgeben kann, wobei der Ladeimpulserzeugungsabschnitt einen Magnetisierungsabschnitt mit ausgerichteten magnetischen Domänen beinhaltet, der durch ein sich veränderndes (externes) Magnetfeld derart kontaktlos beeinflussbar ist, dass in ihm ab Erreichen einer bestimmten Feldstärke (Amplitude) eine zur Erzeugung des Ladeimpulses führende über den Magnetisierungsabschnitt laufende Ummagnetisierungswelle, hervorgerufen durch die fortlaufend umgepolten magnetischen Domänen, auftritt.*

Das genannte externe bzw. extern erzeugte Magnetfeld wird bevorzugt durch das im Folgenden noch erläuterte erfindungsgemäße Ladegerät erzeugt.

Der Magnetisierungsabschnitt des Ladeimpulserzeugungsabschnittes weist gleich ausgerichtete magnetische Domänen auf, die gemeinsam durch das sich verändernde extern erzeugte Magnetfeld beeinflusst werden können. Wenn das extern erzeugte Magnetfeld in einem bestimmten Bereich des Magnetisierungsabschnittes eine bestimmte Amplitude bzw. Feldstärke, die in der Größenordnung weniger Millitesla (kleiner gleich 10mT) liegt, erreicht, magnetisieren sich die Domänen in diesem Bereich um (Umklappen der sog. Weiß'schen Bezirke), wodurch die genannte Ummagnetisierungswelle über den Magnetisierungsabschnitt zu laufen beginnt, so wie es z. B. bei einem im Folgenden noch erwähnten Wiegand- oder Impulsdraht der Fall ist.

Die Form des Magnetisierungsabschnittes ist beliebig.

Wenn die Stärke des extern erzeugten Magnetfeldes beispielsweise an einem Ende des Magnetisierungsabschnittes die bestimmte Amplitude bzw. Stärke erreicht, beginnt die Ummagnetisierungswelle an diesem Ende des Magnetisierungsabschnittes zu laufen, bis sie das andere Ende des Magnetisierungsabschnittes erreicht. Physikalisch gesehen handelt es sich bei der so auftretenden Ummagnetisierungswelle um im Wesentlichen eine Blochwand, die über den Magnetisierungsabschnitt läuft.

Die Ummagnetisierung des Magnetisierungsabschnitts wird zur Erzeugung des Ladeimpuls ausgenutzt, beispielsweise durch Induktion.

An dieser Stelle sei ausdrücklich erwähnt, dass die Größe (Amplitude) und die Geschwindigkeit der Ummagnetisierungswelle nicht oder nur unwesentlich von der Frequenz des sich verändernden extern erzeugten Magnetfelds abhängt, sondern vorwiegend von den Materialdaten des Magnetisierungsabschnittes. Der Auslösezeitpunkt der Ummagnetisierungswelle hängt davon ab, wann das sich verändernde, extern erzeugte Magnetfeld die genannte Amplitude bzw. Feldstärke erreicht, wobei der Gradient der Änderung des Magnetfeldes bzw. die entsprechende Frequenz keine Rolle spielt. Wird die notwendige Stärke (Amplitude) des Magnetfelds erreicht, beginnt die Blochwand bzw. die Ummagnetisierungswelle zu laufen.

Die Umpolfrequenz bzw. die Veränderung des Magnetfelds spielt nur insoweit eine wenngleich untergeordnete Rolle, weil diese nur über die Anzahl der Initiierungen der Ummagnetisierungswelle Auskunft gibt bzw. sie zeigt lediglich an, wie oft die Ummagnetisierungswelle initiiert wird und damit wie oft ein Ladeimpuls erzeugt wird.

*Der erfindungsgemäße elektronische Schrittmacher ist bevorzugt so ausgestaltet, dass der Ladeimpulserzeugungsabschnitt mindestens eine Spule aufweist, die zu dem Magnetisierungsabschnitt derart räumlich angeordnet ist, dass sie bei Auftreten der Ummagnetisierungswelle einen zu dem Ladeimpuls führenden Spannungsimpuls erzeugt.*

Die räumliche Anordnung kann dergestalt sein, dass die Spule um den Magnetisierungsabschnitt gewickelt ist, insbesondere diesen axial umschließend.

Spulen aus elektrisch leitenden Materialen sind bekanntermaßen Induktivitäten. Die Ummagnetisierungswelle führt dazu, dass die Spule aufgrund ihrer induktiven Eigenschaften den zu dem Ladeimpuls führenden Spannungsimpuls erzeugt.

Pro Umpolung des sich verändernden Magnetfelds erzeugt die Spule folglich einen Spannungsimpuls bestimmter Höhe (unabhängig davon, wie schnell sich bzw. mit welcher Frequenz sich das Magnetfeld ändert). Der Magnetisierungsabschnitt und die Spule können beispielsweise so dimensioniert sein, dass der zu dem Ladeimpuls führende Spannungsimpuls in seiner Amplitude 10V und mehr beträgt.

Die von der Spule erzeugten Spannungsimpulse haben alternierend umgekehrte Polaritäten. Zur Ausnutzung aller Spannungsimpulse beinhaltet der Ladeimpulserzeugungsabschnitt eine Ladeelektronik, die die Spannungsimpulse mittels eines Gleichrichters bevorzugt gleichrichtet und/oder in einem Kondensator zwischenspeichert.

Allgemein ausgedrückt besteht der Vorteil der Erfindung darin, dass ein Teil der magnetischen Energie des sich verändernden Magnetfelds in dem Magnetisierungsabschnitt zunächst kumuliert und dann in Form der sich bewegenden Ummagnetisierungswelle quasi schlagartig freigesetzt wird. Die Induktion und damit das Entstehen der elektrischen Spannung findet folglich erst zu diesem Zeitpunkt an/in der Spule statt. D.h. die kontaktlose Energieübertragung beruht nicht darauf, dass das sich verändernde Magnetfeld unmittelbar zur Spannungsinduktion in der Spule verwendet wird, sondern die entsprechende Energie des Magnetfeldes wird in dem Magnetisierungsabschnitt zwischengespeichert und bei Initiierung der Ummagnetisierungswelle quasi schlagartig freigesetzt. Aus diesem Grund kann die Umpolfrequenz so angepasst werden, dass Energie problemlos durch ein aus Metall gebildetes Gehäuse oder Hülle hindurch übertragen werden kann. Erfindungsgemäß handelt es sich um ein Verfahren der indirekten Induktion, d.h. die vom Strom der Primärspule erzeugte Änderung des Magnetflusses führt nicht ausschließlich wie bei der direkten Induktion in der Sekundärspule unmittelbar zu einer Spannung, sondern ein Teil von diesem Fluss wird zunächst in dem Magnetisierungsabschnitt zwischengespeichert. Bei einer bestimmten Feldstärke stimuliert dann der Fluss den Magnetisierungsabschnitt zur Erzeugung einer magnetischen Stoßwelle bestimmter Polarität (Ummagnetisierungswelle) und damit indirekt in der Sekundärspule zu einem Spannungsimpuls bestimmer Polarität mit wesentlich höherer Amplitude. Die Spule des Ladeimpulserzeugungsabschnitts fungiert dabei als die genannte Sekundärspule, die bei Auftreten der Ummagnetisierungswelle den Spannungsimpuls erzeugt. Die genannte Primärspule sitzt beispielsweise in dem im Folgenden noch erläuterten erfindungsgemäßen Ladegerät.

In diesem Punkt unterscheidet sich die Erfindung erheblich von bekannten kontaktlosen Ladevorgängen für Akkumulatoren, die ein elektromagnetisches Wechselfeld zur Energieübertragung ausnutzen. Ein solches elektromagnetisches Wechselfeld hoher Frequenz ist bei zu implantierenden Schrittmachern kaum oder nur sehr schlecht einsetzbar, weil die Eindringtiefe des elektromagnetischen Wechselfelds in den Körper des Lebewesens und vor allem in ein Metallgehäuse aufgrund von auftretenden Effekten, wie beispielsweise dem Skin-Effekt, zu gering ist.

*Der Magnetisierungsabschnitt des erfindungsgemäßen elektronischen Schrittmachers ist bevorzugt durch eine spezielle, z.B. mechanische, Bearbeitung derart aufgebaut, dass die magnetischen Domänen des Magnetisierungsabschnittes gleich ausgerichtet sind. Der Magnetisierungsabschnitt des erfindungsgemäßen elektronischen Schrittmachers weist bevorzugt einen hartmagnetischen Schalenbereich auf, der einen weichmagnetischen Kernbereich umschließt.*

Der hartmagnetische Schalenbereich entsteht beispielsweise bei der Bearbeitung und Herstellung des Magnetisierungsabschnitts. Ein bevorzugtes Material für den Magnetisierungsabschnitts ist Vicalloy, das beispielsweise in Kaltumformschritten zur Ausrichtung der magnetischen Domänen bearbeitet wird.

*Bevorzugt ist der Magnetisierungsabschnitt mindestens ein Impulsdraht oder ein Wieganddraht. Der Magnetisierungsabschnitt kann auch eine Vielzahl von Impulsdrähten oder eine Vielzahl von Wieganddrähten oder eine Kombination aus mindestens einem Impulsdraht und einem Wieganddraht aufweisen.*

*Die Anzahl der Spulen ist auch nicht auf eine einzige beschränkt. Jedem der Drähte könnte eine eigene Spule zugeordnet sein oder alternativ kann eine Vielzahl der Drähte von einer oder mehreren Spulen umgeben sein. Bevorzugt wird die Spule oder werden die Spulen um einen oder mehrere der Drähte gewickelt.*

In diesem Fall bilden die Spulen jeweils eine Sekundärspule des erläuterten Verfahrens indirekter Induktion, zu der Energie mittelbar über den Magnetisierungsabschnitt von der Primärspule, die bevorzugt in dem noch zu erläuternden Ladegerät sitzt, übertragen wird.

*Bevorzugt ist der erfindungsgemäße elektronische Schrittmacher so ausgebildet, dass die Elektronik zusammen mit dem Energiespeicher und dem Ladeimpulserzeugungsabschnitt von einer Hülle bzw. einem Gehäuse vollständig umgeben ist, die*/*das aus einem Material gebildet ist, das von dem Körper des Lebewesens nicht abgestoßen wird. Das Material ist bevorzugt ein nicht-ferromagnetisches Metall, insbesondere Titan, oder eine, insbesondere Titan umfassende, Metalllegierung.* Alternative Metalle sind Edelstähle. Die elektrische Leitfähigkeit der genannten Materialien ist von Bedeutung, um hochfrequente Störfelder, die die Funktion des Schrittmachers, beispielsweise des Herzschrittmachers, beeinträchtigen können, zu dämpfen.

Auch kann das elektrisch leitende Gehäuse zum Beispiel als Massekontakt notwendig sein bzw. dienen, um den Stromkreis über die Elektrode(n) zu schließen. Der Massekontakt kann alternativ beispielsweise durch einen elektrisch leitenden Abschnitt, der auf einer Außenoberfläche eines unter Umständen nicht vollständig leitfähig ausgebildeten Gehäuses freiliegt, gebildet sein. Weiterhin alternativ kann der Massekontakt eine gesonderte Masse(kabel)elektrode sein.

Der Elektrodenabschnitt und/oder der Massekontakt ist beispielsweise lösbar an der Hülle bzw. dem Gehäuse befestigt bzw. durchläuft diese und ist innerhalb der Hülle/Gehäuse mit der Elektronik verbunden.

Wie im Vorhergehenden beschrieben wurde, können die Elektroden und/oder der Massekontakt Kabelelektroden oder auf der Außenoberfläche gebildete Elektroden sein. Die Außenoberfläche ist insbesondere eine Außenoberfläche der Hülle/des Gehäuses.

Insgesamt kann die Hülle/das Gehäuse zusammen mit den aufgenommenen Elementen, dem entsprechenden Elektrodenabschnitt und dem Massekontakt so klein ausgebildet sein, dass der Schrittmacher beispielsweise in der Ausführungsform als Herzschrittmacher in das Herz, beispielsweise direkt in den Herzmuskel, implantiert oder auf einer Außenseite des Herz angebracht werden kann.

Dieses gegenüber dem Stand der Technik enorme Miniaturisierungspotential begründet sich darin, dass der erläuterte Energiespeicher des Schrittmachers aufgrund der leichten Wiederaufladung nicht für eine mehrere Jahre umfassende Laufzeit ausgelegt/dimensioniert werden muss, sondern stark verkleinert sein kann. Dies reduziert gleichzeitig das Gefahrenpotential, beispielsweise bei Auftreten eines Kurzschlusses im Energiespeicher.

Die bereits erwähnte Tatsache, dass der Magnetisierungsabschnitt die magnetische Energie kumuliert und erst die Freisetzung in Form der Ummagnetisierungswelle zu dem Entstehen der induzierten elektrischen Spannung bzw. dem Spannungsimpuls innerhalb des Gehäuses bzw. der Hülle führt, schafft Freiräume für die Wahl des bevorzugt nicht-ferromagnetischen Materials der genannten Hülle bzw. des Gehäuses, weil keine unmittelbaren Anforderungen an Übertragungsfrequenzen gestellt werden müssen.

*Bevorzugt beinhaltet der Ladeimpulserzeugungsabschnitt des erfindungsgemäßen Schrittmachers in einer Richtung, in der die mindestens eine Spule gewickelt ist oder in der die Spulen gewickelt sind, an mindestens einem Endabschnitt des Magnetisierungsabschnittes eine magnetische Sammellinse zur Bündelung und Führung des sich verändernden, extern erzeugten Magnetfeldes auf den Magnetisierungsabschnitt.*

Die genannte Richtung entspricht der Längsrichtung der Spule bzw. der Spulen, in der sie gewickelt ist bzw. sind. Bevorzugt sind an beiden Endabschnitten des Magnetisierungsabschnitts jeweils mindestens eine magnetische Sammellinse angeordnet, die das sich verändernde, extern erzeugte Magnetfeld auf dem Magnetisierungsabschnitt bündeln bzw. zu diesem leiten.

Alternativ zur Verwendung eigenständiger Sammellinse(n) besteht auch die Möglichkeit, dass die Hülle bzw. das Gehäuse gezielt teilweise oder abschnittsweise aus einem ferromagnetischen Material gebildet ist oder teilweise oder abschnittsweise mit einem solchen beschichtet ist und, z.B. durch eine Teilung in zwei getrennte Hälften so aufgebaut ist, dass es die Funktion der magnetischen Sammellinsen direkt übernimmt. Durch die dann größere Ausgestaltung der Sammellinsen kann das vom Ladegerät zu erzeugende Magnetfeld weiter reduziert werden.

*Die mindestens eine magnetische Sammellinse des elektronischen Schrittmachers ist bevorzugt aus einem ferromagnetischen Metall gebildet, welches das extern erzeugte Magnetfeld für den Magnetisierungsabschnitt bündelt.*

Die magnetische(n) Sammellinse(n) ist/sind beispielsweise aus Ferrit ausgebildet und haben beispielsweise die Form eines Hohlzylinders, dessen Achse in Richtung des jeweiligen Endabschnitts des Magnetisierungsabschnittes weist.

Der Magnetisierungsabschnitt ist bevorzugt in den Hohlzylinder eingeführt.

Die Verwendung der magnetischen Sammellinse(n) ermöglicht beispielsweise, dass das im Folgenden noch zu erläuternde Ladegerät ein schwächeres Magnetfeld erzeugen muss.

*Die Elektronik des erfindungsgemäßen elektronischen Schrittmachers beinhaltet bevorzugt keine Elemente aus ferromagnetischen Materialien und*/*oder der Ladeimpulserzeugungsabschnitt des erfindungsgemäßen elektronischen Schrittmachers beinhaltet bis auf den Magnetisierungsabschnitt und, wenn bevorzugt vorgesehen, die mindestens eine magnetische Sammellinse keine Elemente aus ferromagnetischen Materialen.*

Diese Ausgestaltung des erfindungsgemäßen elektronischen Schrittmachers ist dahingehend vorteilhaft, dass die nicht aus ferromagnetischen Materialien gebildeten Elemente durch das extern erzeugte Magnetfeld nicht beeinträchtigt bzw. gestört werden.

*Weiterhin ist die Elektronik des erfindungsgemäßen elektronischen Schrittmachers eingerichtet, ein Signal auszusenden, welches die Qualität des Ladeimpulses anzeigt.*

Das genannte Signal kann beispielsweise ein niederfrequentes Signal sein, das den Köper des Lebewesens und, wenn keine Antenne hierfür vorgesehen ist, die Hülle bzw. das Gehäuse des erfindungsgemäßen Schrittmachers durchdringt.

Die Qualität des Ladeimpulses bestimmt sich beispielsweise daran, wie stark der Ladeimpuls ist. Das die Qualität des Ladeimpulses angebende Signal kann beispielsweise ein binäres Signal sein, das einen OK-Zustand einnimmt, wenn der Ladeimpuls einen Schwellenwert übersteigt, und einen NG-Zustand einnimmt, wenn der Ladeimpuls den Schwellenwert nicht übersteigt. Alternativ kann das genannte Signal auch die genaue Stärke des Ladeimpulses angeben.

*Die Erfindung betrifft ebenfalls ein Ladegerät für einen elektronischen Schrittmacher, wobei das Ladegerät eingerichtet ist, ein sich mit einer Umpolfrequenz und Amplitude veränderndes Magnetfeld zu erzeugen. Das Ladegerät wird bei bestimmungsgemäßer Verwendung vorübergehend auf einer Körperoberfläche des Lebewesens oder in der Nähe der Körperoberfläche des Lebewesens derart angeordnet, dass das Magnetfeld in den Körper und den erfindungsgemäßen implantierten elektronischen Schrittmacher zur Beeinflussung des Ladeimpulserzeugungsabschnittes eindringt.*

*Die Umpolfrequenz liegt bevorzugt in einem Bereich von*
- *X bis 10kHz, wobei X>0 und X*>*= 0,1kHz, 0,2kHz, 0,3kHz,...,4,9kHz,...,9,9kHz ist.*

Das Ladegerät beinhaltet beispielsweise eine oder eine Vielzahl von Spulen, die in dem im Vorhergehenden erwähnten Verfahren indirekter Induktion als die Primärspule(n) fungieren. Bevorzugt ist ein Kern, beispielsweise aus Ferrit, in die Spule(n) eingesetzt.

Das Ladegerät erzeugt bestimmungsgemäß einen Stromfluss durch die Spule(n) für den Aufbau eines sich verändernden elektromagnetischen Feldes, das sich mit der erwähnten Umpolfrequenz umpolt. Wenn das Ladegerät auf der Körperoberfläche bzw. in deren Nähe angeordnet ist, kann das Wechselfeld in den Körper des Lebewesens und einen dort angeordneten erfindungsgemäßen Schrittmacher, beispielsweise einen Herzschrittmacher, eindringen. Der magnetische Anteil des sich verändernden elektromagnetischen Feldes bildet das im Vorhergehenden erläuterte, extern erzeugte Magnetfeld, das den Magnetisierungsabschnitt zur Initiierung der Ummagnetisierungswelle beeinflusst.

Die Stärke und/oder die Umpolfrequenz des elektromagnetischen Wechselfeldes können bevorzugt in dem Ladegerät gesteuert werden. Dies ist dahingehend vorteilhaft, dass das Ladegerät in Abhängigkeit von der Lage des Schrittmachers innerhalb des Körpers bzw. in Abhängigkeit von der notwendigen Eindringtiefe eingestellt werden kann.

*Das erfindungsgemäße Ladegerät beinhaltet eine Vielzahl von Spulen zur Erzeugung des sich verändernden Magnetfeldes, wobei die Vielzahl von Spulen auf Basis des die Qualität des Ladeimpulses anzeigenden Signals für eine Optimierung des Ladeimpulses entsprechend angesteuert werden.*

Die Spulen der Vielzahl von Spulen sind bevorzugt so räumlich angeordnet, dass das Ladegerät durch die Ansteuerung der Spulen die Ausrichtung des erzeugten Magnetfeldes ändern kann. Dies hat den Vorteil, dass das Ladegerät die Ausrichtung des Magnetfeldes unter Berücksichtigung des die Qualität des Ladeimpulses angebenden Signals ändern kann, um die Qualität der Ladeimpulse zu verbessern bzw. zu optimieren.

Das Ladegerät ist bevorzugt eingerichtet, die Spulen zur Ausrichtung des Magnetfeldes automatisch anzusteuern.

Im Folgenden wird eine bevorzugte Ausführungsform der Erfindung unter Bezug auf die beigefügte Figur erläutert.
**Figur 1** zeigt eine schematische Darstellung eines erfindungsgemäßen Implantat-Schrittmachers;
**Figur 2** zeigt eine schematische Darstellung eines entsprechenden Ladegerätes;
**Figur 3** zeigt eine alternative Ausgestaltung des in Figur 2 gezeigten Ladegerätes mit einer Vielzahl von Spulen.

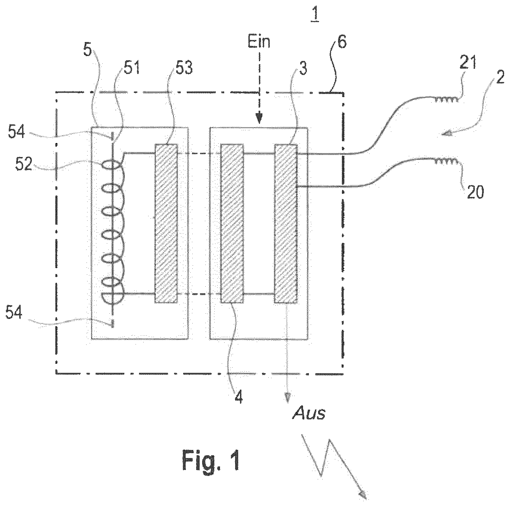

Figur 1 zeigt den schematischen Aufbau eines erfindungsgemäßen elektronischen Schrittmachers 1.

Der elektronische Schrittmacher 1 ist in dieser bevorzugten Ausführungsform ein Herzschrittmacher, der bestimmungsgemäß vollständig in den menschlichen Körper implantiert wird. Die Erfindung ist allerdings nicht auf einen Herzschrittmacher beschränkt. Bei dem elektronischen Schrittmacher 1 kann es sich beispielsweise auch um einen Gehirnschrittmacher oder einen Darmschrittmacher handeln.

Der Herzschrittmacher 1 beinhaltet einen Elektrodenabschnitt 2, der in dieser bevorzugten Ausführungsform zwei Elektroden 20, 21 beinhaltet. Der Herzschrittmacher 1 ist demgemäß ein Zweikammer-Herzschrittmacher, der eingerichtet ist, über die Elektroden 20, 21 einen Vorhof (Atrium) und eine Herzkammer (Ventrikel) einer Herzseite zu stimulieren. Hierfür wird eine der Elektroden bestimmungsgemäß mit dem Vorhof verbunden und die andere der Elektroden mit der entsprechenden Herzkammer.

Die Ausgestaltung als Zweikammer-Herzschrittmacher ist auch lediglich bevorzugt. Alternativ kann der Herzschrittmacher auch ein Einkammer- o- der Dreikammer-Herzschrittmacher sein, wobei er dann die hierfür notwendige Anzahl von Elektroden besitzt.

Der Elektrodenabschnitt 2 ist mit einer Elektronik 3 verbunden. Die Elektronik 3 ist eingerichtet, die notwendigen Funktionen des Herzschrittmachers zu übernehmen. Die Elektronik 3 erhält ein Eingangssignal Ein bzw. erhält Körperdaten, über das/die der Herzschrittmacher bzw. die Elektronik 3 erkennen kann, ob die zu überwachende und steuernde Körperfunktion (Herzschlag) stimuliert bzw. gesteuert werden muss. Wenn die Elektronik 3 beispielsweise erkennt, dass nach Ablauf eines bestimmten Zeitintervalls kein Herzschlag vorliegt, erzeugt sie einen Impuls bzw. Stimulationsimpuls (Strom- und/oder Spannungsimpuls), den sie zur Anregung des Herz über den Elektrodenabschnitt 2 an den Vorhof und/oder die Herzkammer abgibt.

Die Elektronik 3 ist bevorzugt so eingerichtet, dass sie nur im Bedarfsfall den Impuls erzeugt und das Herz anregt.

Der Herzschrittmacher 1 beinhaltet zur Versorgung der Elektronik 3 einen elektrischen Energiespeicher 4, beispielsweise einen Akkumulator, der mit der Elektronik 3 elektrisch verbunden ist. Der Energiespeicher 4 ist beispielsweise ein Lithium-Ionen-Akkumulator, der wieder aufgeladen werden kann. Eine andere Lösung für einen Energiespeicher wäre z.B. ein Kondensator mit extrem geringer Selbstentladung (z.B. Gold-Cap).

Als erfindungswesentliches Element weist der Herzschrittmacher 1 einen Ladeimpulserzeugungsabschnitt 5 auf, über den der Energiespeicher 4 wieder aufgeladen werden kann. Der Ladeimpulserzeugungsabschnitt 5 ermöglicht ein kontaktloses Laden des Energiespeichers 4.

Der Ladeimpulserzeugungsabschnitt 5 beinhaltet als wesentliche Elemente mindestens einen Impulsdraht oder Wieganddraht 51, der von einer Spule 52 axial umschlossen wird bzw. um den die Spule 52 gewickelt ist, und eine Ladeelektronik 53.

Der Impulsdraht oder Wieganddraht 51 bildet einen Magnetisierungsabschnitt, der durch ein sich veränderndes, extern erzeugtes Magnetfeld beeinflusst werden kann. Bevorzugt kann der Magnetisierungsabschnitt 51 eine Vielzahl von Impulsdrähten und/oder Wieganddrähten beinhalten, wobei jeder der Drähte oder eine Vielzahl der Drähte von einer oder mehreren Spulen umschlossen sein kann.

Das sich verändernde Magnetfeld wird beispielsweise durch ein im Folgenden noch erläutertes Ladegerät erzeugt.

Der Magnetisierungsabschnitt 51 weist gleichmäßig ausgerichtete magnetische Domänen auf, die bei Veränderung des Magnetfeldes ab einer bestimmten Amplitude bzw. Feldstärke in der Größenordnung weniger MilliTesla beginnen, sich umzumagnetisieren (umzuklappen). Dies führt dazu, dass physikalisch gesehen eine Ummagnetisierungswelle (Blochwand) über den Magnetisierungsabschnitt läuft. In der Literatur wird dieses Ereignis auch als großer Barkhausen-Sprung bezeichnet.

Die Größe und Geschwindigkeit der Ummagnetisierungswelle ist unabhängig von der Frequenz (Umpolfrequenz), mit der sich das extern erzeugte Magnetfeld ändert. Die über den Magnetisierungsabschnitt laufende Ummagnetisierungswelle erzeugt in der/den um den Magnetisierungsabschnitt 51 gewickelte(n) Spule(n) 52 einen Spannungsimpuls.

Der Spannungsimpuls wird bevorzugt von der Ladeelektronik 53 verarbeitet. Die Ladeelektronik 53 beinhaltet beispielsweise einen Gleichrichter zur Gleichrichtung der Spannungsimpulse der Spule(n), die alternierend mit jeweils umgekehrter Polarität erzeugt werden, und bevorzugt einen Kondensator (beispielsweise auch ein Gold-Cap) zur Zwischenspeicherung elektrischer Energie.

Die Ladeelektronik 53 gibt letztendlich einen Ladeimpuls an den Energiespeicher 4 aus, wodurch dieser aufgeladen wird.

Die Elektronik 3 ist ausgestaltet, ein Signal *Aus* auszugeben, das die Qualität des von der Ladeelektronik 53 ausgegebenen Ladeimpulses angibt. Beispielsweise erfasst die Elektronik 3 die Stärke des Ladeimpulses und generiert hierauf beruhend das Signal *Aus.* Die Ausgabe des Signals *Aus* erfolgt beispielsweise als niederfrequentes Funksignal. Das Signal *Aus* wird von dem im Folgenden noch erläuterten Ladegerät verarbeitet.

Die Elektronik 3, der Energiespeicher 4 und der Ladeimpulserzeugungsabschnitt 5 sind zusammen in ein Gehäuse 6 aufgenommen und werden von diesem vollständig umschlossen. Das Gehäuse 6 ist bevorzugt aus Titan bzw. einer entsprechenden Legierung aufgebaut und eignet sich deshalb hervorragend zur Implantation in den menschlichen Körper, weil keine Abstoßungsreaktionen auftreten und hält als metallischer Körper hochfrequente Störfelder fern. Ein metallischer Körper kann zudem als die für den Stromimpuls notwendige Masseelektrode bzw. als das Massepotential verwendet werden, was z.B. bei einem Glaskörper nicht möglich wäre. Im Falle eines nicht elektrisch leitenden Gehäuses, wie beispielsweise bei dem genannten Glaskörper, kann das Massepotential durch einen elektrisch leitenden Abschnitt, der auf einer Außenoberfläche des Gehäuses freiliegt, oder eine gesonderte Masse(kabel)elektrode gebildet sein.

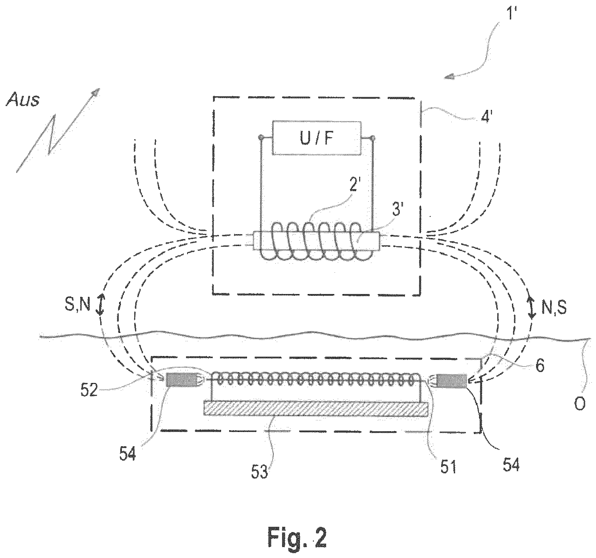

Figur 2 zeigt schematisch den Aufbau eines erfindungsgemäßen Ladegerätes 1' das zum Wiederaufladen des elektronischen Schrittmachers 1 dient.

Erfindungsgemäß nützt die Erfindung allgemein ein Verfahren indirekter Induktion um den Energiespeicher 4 wieder aufzuladen, d.h. die Energie wird nicht wie bei der direkten Induktion direkt von einer Primär- unverzögert zu einer Sekundärspule übertragen (Transformatorprinzip), sondern indirekt von einer Primärspule, die in dem folgend erläuterten Ladegerät sitzt, erst auf den die Energie speichernden Magnetisierungsabschnitt 51 und von dort verzögert zu der/den den Magnetisierungsabschnitt 51 umschließenden Spulen 52.

Das Ladegerät 1' beinhaltet zum Beispiel eine Spule (Primärspule) 2', deren Strom bzw. Spannung in Amplitude und Frequenz geregelt werden kann. Bevorzugt hat die Spule 2' zur Feldverstärkung einen ferromagnetischen Kern 3', z.B. aus Ferrit.

Ein Gehäuse 4' nimmt die entsprechenden Komponenten des Ladegerätes 1' auf. Das Gehäuse 4' wird bei bestimmungsgemäßer Verwendung zweitweise in der Nähe oder auf einer Oberfläche O des menschlichen Körpers so angeordnet, dass das von der Primärspule 2' im Ladegerät erzeugte externe Magnetfeld den Magnetisierungsabschnitt 51 erreicht.

Bei Betrieb des Ladegeräts 1' erzeugt dieses über die Primärspule 2' ein sich veränderndes Magnetfeld, das einen Anteil des von der Primärspule 2' erzeugten elektromagnetischen Feldes bildet. Das erzeugte sich verändernde Magnetfeld polt sich mit einer bestimmten Umpolfrequenz um und erreicht den Magnetisierungsabschnitt 51. Jede Umpolung führt ab Erreichen einer bestimmten Feldstärke zu der Initiierung der Ummagnetisierungswelle, wobei die Spule 52, die in dem erwähnten Verfahren der indirekten Induktion die Sekundärspule bildet, alternierend positive und negative Spannungsimpulse erzeugt. Die Spannungsimpulse werden, wie bereits erläutert, von der Ladeelektronik 53 verarbeitet, sodass die Ladeelektronik 53 letztendlich den Ladeimpuls an den Energiespeicher 4 abgibt.

Ein wesentlicher Punkt der Erfindung ist, wie bereits erwähnt, dass die kontaktlose Energieübertragung nicht darauf beruht, dass das sich verändernde Magnetfeld, das von dem Ladegerät 1' erzeugt wird, direkt zur Spannungsinduktion in der Sekundärspule 52 verwendet wird, sondern indirekt, indem die entsprechende Energie des Magnetfeldes in dem Magnetisierungsabschnitt 51 zwischengespeichert und dann bei Initiierung der Ummagnetisierungswelle quasi schlagartig freigesetzt wird, wodurch der Spannungsimpuls in der Sekundärspule 52 durch Induktion erzeugt wird. Aus diesem Grund kann die Umpolfrequenz so angepasst werden, dass die Energie problemlos auch durch das aus Metall gebildete Gehäuse 6 hindurch übertragen werden kann.

Die Stärke und/oder Umpolfrequenz des von der Primärspule 2' erzeugten Magnetfeldes bzw. elektromagnetischen Feldes kann in dem Ladegerät 1' gesteuert werden, um das Wiederaufladen des Energiespeichers 4 an die spezifische Lage des Schrittmachers 1 in dem Körper des Lebewesens bzw. die notwendige Eindringtiefe anzupassen und die Ladezeit zu minimieren. Die Steuerung der Stärke und/oder Umpolfrequenz des von der Primärspule 2' erzeugten Magnetfeldes bzw. elektromagnetischen Feldes erfolgt in dem Ladegerät 1' bevorzugt auf Basis des Signals *Aus,* das von dem Schrittmacher ausgesendet wird. Hierfür beinhaltet das Ladegerät 1`entsprechende Empfangseigenschaften, um das (Funk)signal *Aus* zu empfangen.

Bevorzugt beinhaltet der Schrittmacher 1 in der Längsrichtung der Spule(n) 52 an Endabschnitten des Magnetisierungsabschnittes 51 magnetische Sammellinsen 54 zur Bündelung des sich verändernden Magnetfeldes. Die magnetischen Sammellinsen 54 können bevorzugt die Form eines Hohlzylinders haben, in den der Impulsdraht/Wieganddraht bzw. die Impulsdrähte/Wieganddrähte eingeführt sind.

Alternativ oder zusätzlich zu dem magnetischen Sammellinsen 54 kann das Gehäuse 6 des Schrittmachers 1 aus zwei zusammengesetzten Gehäuseabschnitten aufgebaut sein. Die Gehäuseabschnitte können aus ferromagnetischen Metallen ausgebildet sein oder mit solchen beschichtet sein, wobei die Ausrichtung des Ladeimpulserzeugungsabschnittes 5 innerhalb des Gehäuses 6 so gewählt ist, dass die Gehäuseabschnitte als zusätzliche oder auch alleinige Sammellinsen 54 wirken.

Pro Umpolung des sich verändernden externen Magnetfeldes wird die Ummagnetisierungswelle, die über den Magnetisierungsabschnitt 51 läuft initiiert und letztendlich einer der Ladeimpulse zum Wiederaufladen des Energiespeichers 4 erzeugt.

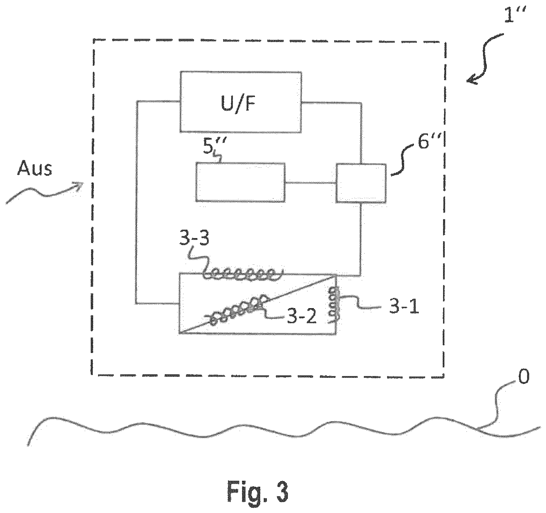

Figur 3 zeigt eine alternative Ausgestaltung eines erfindungsgemäßen Ladegeräts 1".

Der Schrittmacher ist wie in Figur 2 angeordnet und in Figur 3 nicht mehr gezeigt.

Das gezeigte Ladegerät 1" unterscheidet sich von dem aus Figur 2 lediglich dadurch, dass eine Vielzahl von Spulen 3-1, 3-2, 3-3, die jeweils als die genannte Primärspule fungieren, vorgesehen ist. Das Ladegerät 1" beinhaltet bevorzugt eine Elektronik 5" und einen Multiplexer 6". Die Elektronik 5" ist dazu eingerichtet, den Multiplexer 6" anzusteuern und hierdurch festzulegen, welche oder in welcher Kombination die Spulen 3-1, 3-2, 3-3 zur Erzeugung des Magnetfeldes verwendet werden. Die Ansteuerung der Spulen 3-1, 3-2, 3-3 beruht auf dem (Funk)signal *Aus* des Schrittmachers 1, das die Qualität des Ladeimpulses angibt.

Die Spulen 3-1, 3-2, 3-3 sind räumlich unterschiedlich angeordnet, wodurch die Orientierung des Magnetfeldes zur Verbesserung und Optimierung des Ladeimpulses geändert werden kann.

Jeder der Spulen 3-1, 3-2, 3-3 beinhaltet bevorzugt einen Kern wie er in Figur 2 gezeigt und in die Spule 2' eingeführt ist.

## Patentansprüche

1. Elektronischer Schrittmacher (1) zur Implantation in einen Körper eines Lebewesens und zur Steuerung einer Körperfunktion, wobei der Schrittmacher aufweist:
einen Elektrodenabschnitt (2), der bestimmungsgemäß an einem Körperabschnitt zu befestigen ist;
eine mit dem Elektrodenabschnitt (2) verbundene Elektronik (3), die eingerichtet ist, einen Impuls, insbesondere einen Spannungsimpuls, zu erzeugen und diesen über den Elektrodenabschnitt (2) an den Körperabschnitt zur Steuerung der Körperfunktion abzugeben;
einen Energiespeicher (4) zur Versorgung der Elektronik (3) mit elektrischer Energie, der nach Entladung mit elektrischer Energie wieder aufgeladen werden kann; und
einen mit dem Energiespeicher (4) elektrisch verbundenen Ladeimpulserzeugungsabschnitt (5), der derart eingerichtet ist, dass er an den Energiespeicher (4) einen Ladeimpuls zum Wiederaufladen des Energiespeichers (4) abgeben kann;
wobei
der Ladeimpulserzeugungsabschnitt (5) einen Magnetisierungsabschnitt (51) mit ausgerichteten magnetischen Domänen beinhaltet, der durch ein sich veränderndes Magnetfeld derart kontaktlos beeinflussbar ist, dass in ihm ab Erreichen einer bestimmten Feldstärke eine zur Erzeugung des Ladeimpulses führende über den Magnetisierungsabschnitt (51) laufende Ummagnetisierungswelle, hervorgerufen durch die fortlaufend umgepolten magnetischen Domänen, auftritt; und
die Elektronik (3) eingerichtet ist, ein Signal auszusenden, welches die Qualität des Ladeimpulses anzeigt.

2. Elektronischer Schrittmacher (1) gemäß Patentanspruch 1, wobei der Ladeimpulserzeugungsabschnitt (5) mindestens eine Spule (52) aufweist, die zu dem Magnetisierungsabschnitt (51) derart räumlich, bevorzugt um den Magnetisierungsabschnitt (51) diesen axial umschließend gewickelt, angeordnet ist, dass sie bei Auftreten der Ummagnetisierungswelle einen zu dem Ladeimpuls führenden Spannungsimpuls erzeugt.

3. Elektronischer Schrittmacher (1) gemäß Patentanspruch 2, wobei der Magnetisierungsabschnitt (51) durch mechanische Bearbeitung derart ausgebildet ist, dass die magnetischen Domänen des Magnetisierungsabschnittes (51) gleich ausgerichtet sind.

4. Elektronischer Schrittmacher (1) gemäß Patentanspruch 3, wobei der Magnetisierungsabschnitt (51) einen hartmagnetischen Schalenbereich aufweist, der einen weichmagnetischen Kernbereich umschließt.

5. Elektronischer Schrittmacher (1) gemäß einem der Patentansprüche 1 bis 3, wobei der Magnetisierungsabschnitt mindestens ein Impulsdraht oder ein Wieganddraht ist.

6. Elektronischer Schrittmacher (1) gemäß Patentanspruch 5, wobei der Magnetisierungsabschnitt (51) eine Vielzahl von Impulsdrähten oder eine Vielzahl von Wieganddrähten oder eine Kombination aus mindestens einem Impulsdraht und einem Wieganddraht aufweist.

7. Elektronischer Schrittmacher (1) gemäß Patentanspruch 6, wobei die Spule (52) um die Vielzahl oder die Kombination von Drähten gewickelt ist, oder
mehrere Spulen (52) vorgesehen sind, die jeweils um mindestens einen der Drähte gewickelt sind.

8. Elektronischer Schrittmacher (1) gemäß einem der Patentansprüche 2 bis 7, wobei der Ladeimpulserzeugungsabschnitt (5) in einer Richtung, in der die mindestens eine Spule (52) gewickelt ist, an mindestens einem Endabschnitt des Magnetisierungsabschnittes (51) eine magnetische Sammellinse (54) zur Bündelung und Führung des sich verändernden Magnetfeldes auf den Magnetisierungsabschnitt (51) aufweist.

9. Elektronischer Schrittmacher (1) gemäß einem der Patentansprüche 1 bis 8, wobei die Elektronik (3) zusammen mit dem Energiespeicher (4) und dem Ladeimpulserzeugungsabschnitt (5) von einer Hülle bzw. einem Gehäuse vollständig umgeben ist, die aus einem Material gebildet ist, das von dem Körper des Lebewesens nicht abgestoßen wird.

10. Elektronischer Schrittmacher (1) gemäß Patentanspruch 9, wobei das Material ein bevorzugt nicht-ferromagnetisches Metall, insbesondere Titan, oder eine, insbesondere Titan umfassende, Metalllegierung ist.

11. Elektronischer Schrittmacher (1) gemäß einem der vorhergehenden Ansprüche 1 bis 10, wobei die Elektronik (3) und/oder der Ladeimpulserzeugungsabschnitt (5) bis auf den Magnetisierungsabschnitt (51) und, wenn bevorzugt vorgesehen, die mindestens eine magnetische Sammellinse (54) keine Elemente aus ferromagnetischen Materialen aufweist.

12. Elektronischer Schrittmacher (1) gemäß einem der Patentansprüche 1 bis 11, wobei der Energiespeicher ein Akkumulator, beispielsweise ein Lithium-Ionen-Akkumulator, ist.

13. Elektronischer Schrittmacher (1) gemäß Patentanspruch 1, wobei der Energiespeicher (4) ein Kondensator ist.

14. Elektronischer Schrittmacher (1) gemäß einem der Patentansprüche 8 bis 13, wobei die mindestens eine magnetische Sammellinse (54) aus einem ferromagnetischen Metall gebildet ist, welches das Magnetfeld für den Magnetisierungsabschnitt (51) bündelt.

15. Ladegerät (1") für einen elektronischen Schrittmacher (1), wobei das Ladegerät (1") eingerichtet ist, ein sich mit einer Umpolfrequenz und bevorzugt Amplitude veränderndes Magnetfeld zu erzeugen, und das Ladegerät (1") bei bestimmungsgemäßer Verwendung auf einer Körperoberfläche (O) des Lebewesens oder in der Nähe der Körperoberfläche (O) des Lebewesens derart angeordnet wird, dass das Magnetfeld in den Körper und einen implantierten elektronischen Schrittmacher (1) gemäß einem der Patentansprüche 1 bis 14 zur Beeinflussung des Ladeimpulserzeugungsabschnittes eindringt, und wobei mehrere Spulen (3-1, ... , 3-3) zur Erzeugung des sich verändernden Magnetfeldes vorgesehen sind, welche auf Basis des die Qualität des Ladeimpulses anzeigenden Signals für eine Optimierung des Ladeimpulses entsprechend angesteuert werden können.

16. Ladegerät (1") gemäß Patentanspruch 15, wobei die Umpolfrequenz in einem Bereich liegt von
• X bis 10kHz, wobei X>0 und X>= 0,1kHz, 0,2kHz, 0,3kHz,...,4,9kHz,..., oder 9,9kHz ist.

## Claims

1. Electronic pacemaker (1) for implantation in a body of a living being and for controlling a bodily function, wherein the pacemaker comprises:
an electrode portion (2) that is, according to its intended purpose, to be attached to a body portion;
an electronics assembly (3) connected with the electrode portion (2), configured to generate an impulse, in particular a voltage impulse, and to emit the same via the electrode portion (2) to the body portion to control the bodily function;
an energy storage (4) to supply the electronics assembly (3) with electrical energy which can be recharged with electrical energy after discharge; and
a charging impulse generation portion (5) electrically connected to the energy storage (4), which is configured to be able to emit a charging impulse to the energy storage (4) for the recharging of the energy storage (4);
wherein
the charging impulse generation portion (5) comprises a magnetization portion (51) with oriented magnetic domains which can be contactlessly influenced by an changing magnetic field so that, when a certain field strength is reached, a remagnetization wave, caused by the continuously reversing magnetic domains, occurs in the magnetization portion (51) which runs across the magnetization portion (51) and leads to the generation of the charging impulse; and
the electronics assembly (3) of the electronic pacemaker is configured to send out a signal that indicates the quality of the charging impulse.

2. The electronic pacemaker (1) according to the claim 1, wherein the charging impulse generation portion (5) comprises at least one coil (52) which is spatially arranged to the magnetization portion (51), preferably surrounding the magnetization portion (51) axially, so that it generates a voltage impulse, which leads to the charging impulse, when the remagnetization wave occurs.

3. Electronic pacemaker (1) according to claim 2, wherein the magnetization portion (51) is configured by mechanical machining so that the magnetic domains of the magnetization portion (51) are equally oriented.

4. Electronic pacemaker (1) according to claim 3, wherein the magnetization portion (51) comprises a magnetically hard shell area which encloses a magnetically soft core area.

5. Electronic pacemaker (1) according to any of claims 1 to 3, wherein the magnetization portion (51) is at least an impulse wire or a Wiegand wire.

6. Electronic pacemaker (1) according to claim 5, wherein the magnetization portion comprises a plurality of impulse wires or a plurality of Wiegand wires or a combination of at least one impulse wire and one Wiegand wire.

7. Electronic pacemaker (1) according to claim 6, wherein the coil (52) is wound around the plurality or the combination of wires, or
several coils (52) are provided which are each wound around at least one of the wires.

8. Electronic pacemaker (1) according to any of claims 2 to 7, wherein the charging impulse generation portion (5) comprises, in a direction in which the at least one coil (52) is wound, a magnetic collecting lens (54) at at least one end portion of the magnetization portion (51) for bundling and guidance of the changing externally generated magnetic field to the magnetization portion (51).

9. Electronic pacemaker (1) according to any of claims 1 to 8, wherein the electronics assembly (3) together with the energy storage (4) and the charging impulse generation portion (5) are fully surrounded by a sleeve or a case which is made from a material that is not rejected by the body of the living being.

10. Electronic pacemaker (1) according to claim 9, wherein the material is preferably a non-ferromagnetic metal, in particular titanium, or a metal alloy comprising titanium in particular.

11. Electronic pacemaker (1) according to any of the preceding claims 1 to 10, wherein the electronics assembly (3) and/or the charging impulse generation portion (5), except for the magnetization portion (51) and, when preferably provided, the at least one magnetic collecting lens (54), does not comprise any elements from ferromagnetic materials.

12. Electronic pacemaker (1) according to any of claims 1 to 11, wherein the energy storage is an accumulator, for example a lithium-ion accumulator.

13. Electronic pacemaker (1) according to claim 1, wherein the energy storage (4) is a capacitor.

14. Electronic pacemaker (1) according to any of claims 8 to 13, wherein the at least one magnetic collecting lens (54) is formed from ferromagnetic metal which bundles the magnetic field for the magnetization portion (51).

15. Charging device (1") for an electronic pacemaker (1), wherein the charging device (1") is configured to generate a magnetic field that changes with a commutation frequency and preferably amplitude, and the charging device (1"), when used as intended, is arranged on a body surface (O) of the living being or close to the body surface (O) of the living being so that the magnetic field penetrates the body and an implanted electronic pacemaker according to any of claims 1 to 14 to influence the charging impulse generation portion, and wherein a plurality of coils (3-1, ... , 3-3) is provided for the generation of the changing magnetic field which coils can be controlled accordingly on the basis of the signal indicating the quality of the charging impulse, for an optimization of the charging impulse.

16. Charging device according to claim 15, wherein the commutation frequency is in a range from
• X to 10kHz, wherein X>0 and X>= 0.1kHz, 0.2kHz, 0.3kHz,..., 4.9kHz,..., 9.9kHz.

## Revendications

1. Stimulateur électronique (1) destiné à être implanté dans le corps d'un être vivant et à contrôler une fonction corporelle, le stimulateur comprenant :
une partie électrode (2) à fixer contre une partie de corps conformément à sa destination ;
une électronique (3) reliée à la partie électrode (2), qui est configurée pour générer une impulsion, en particulier une impulsion de tension, et à délivrer cette dernière à la partie de corps via la partie électrode (2) pour contrôler la fonction corporelle ;
un dispositif de stockage d'énergie (4) pour alimenter l'électronique (3) en énergie électrique, qui peut être rechargé avec de l'énergie électrique après avoir été déchargé ; et
une partie de génération d'impulsions de charge (5) qui est reliée électriquement au dispositif de stockage d'énergie (4) et qui est disposée de telle manière qu'elle soit apte à délivrer au dispositif de stockage d'énergie (4) une impulsion de charge pour recharger le dispositif de stockage d'énergie (4) ;
dans lequel
la partie de génération d'impulsions de charge (5) contient une partie de magnétisation (51) avec des domaines magnétiques alignés qui peut être influencée sans contact par un champ magnétique se modifiant tel que, dès qu'une certaine intensité de champ est atteinte, une onde d'inversion magnétique apparaît en elle, qui est causée par l'inversion continue de la polarité des domaines magnétiques, qui conduit à la génération de l'impulsion de charge et qui passe sur la partie de magnétisation (51) ; et
l'électronique (3) est configurée pour émettre un signal indiquant la qualité de l'impulsion de charge.

2. Stimulateur électronique (1) selon la revendication 1, dans lequel la partie de génération d'impulsions de charge (5) présente au moins une bobine (52) qui est disposée dans l'espace par rapport à la partie de magnétisation (51), de préférence enroulée autour de la partie de magnétisation (51) de manière à l'entourer axialement, de sorte à ce qu'elle génère une impulsion de tension conduisant à l'impulsion de charge lors de l'apparition de l'onde d'inversion magnétique.

3. Stimulateur électronique (1) selon la revendication 2, dans lequel la partie de magnétisation (51) est formée par un traitement mécanique de sorte à ce que les domaines magnétiques de la partie de magnétisation (51) soient alignés de manière égale.

4. Stimulateur électronique (1) selon la revendication 3, dans lequel la partie de magnétisation (51) comprend une région d'enveloppe magnétique dure entourant une région de noyau magnétique douce.

5. Stimulateur électronique (1) selon l'une quelconque des revendications 1 à 3, dans lequel la partie de magnétisation est au moins un fil d'impulsion ou un fil de Wiegand.

6. Stimulateur électronique (1) selon la revendication 5, dans lequel la partie de magnétisation (51) comprend une pluralité de fils d'impulsion ou une pluralité de fils de Wiegand ou une combinaison d'au moins un fil d'impulsion et un fil de Wiegand.

7. Stimulateur électronique (1) selon la revendication 6, dans lequel
la bobine (52) est enroulée autour de la pluralité ou de la combinaison de fils, ou
plusieurs bobines (52) sont prévues, chacune d'entre elles étant enroulée autour d'au moins un des fils.

8. Stimulateur électronique (1) selon l'une quelconque des revendications 2 à 7, dans lequel la partie de génération d'impulsions de charge (5) comporte, par rapport à une direction dans laquelle la au moins une bobine (52) est enroulée, sur au moins une partie d'extrémité de la partie de magnétisation (51), une lentille de convergence magnétique (54) pour faire converger et guider le champ magnétique se modifiant sur la partie de magnétisation (51).

9. Stimulateur électronique (1) selon l'une quelconque des revendications 1 à 8, dans lequel l'électronique (3), ainsi que le dispositif de stockage d'énergie (4) et la partie de génération d'impulsions de charge (5), est complètement entourée par une gaine ou un boîtier, laquelle est formée d'un matériau qui n'est pas rejeté par le corps de l'être vivant.

10. Stimulateur électronique (1) selon la revendication 9, dans lequel le matériau est de préférence un métal non ferromagnétique, en particulier du titane, ou un alliage métallique comprenant en particulier du titane.

11. Stimulateur électronique (1) selon l'une des revendications précédentes 1 à 10, dans lequel l'électronique (3) et/ou la partie de génération d'impulsions de charge (5) ne comporte pas d'éléments en matériaux ferromagnétiques, à l'exception de la partie de magnétisation (51) et, lorsqu'elle est prévue de préférence, de la au moins une lentille de convergence magnétique (54).

12. Stimulateur électronique (1) selon l'une des revendications précédentes 1 à 11, dans lequel le dispositif de stockage d'énergie est un accumulateur, par exemple un accumulateur au lithium-ion.

13. Stimulateur électronique (1) selon la revendication 1, dans lequel le dispositif de stockage d'énergie (4) est un condensateur.

14. Stimulateur électronique (1) selon l'une des revendications précédentes 8 à 13, dans lequel la au moins une lentille de convergence magnétique (54) est formée d'un métal ferromagnétique qui fait converger le champ magnétique à l'usage de la partie de magnétisation (51).

15. Chargeur (1") pour un stimulateur électronique (1), dans lequel le chargeur (1") est configuré pour générer un champ magnétique se modifiant avec une fréquence d'inversion de polarité et de préférence une amplitude, et le chargeur (1") est disposé, lors d'une utilisation conforme à sa destination, sur une surface de corps (O) de l'être vivant ou à proximité de la surface de corps (O) de l'être vivant de sorte à ce que le champ magnétique pénètre dans le corps et dans un stimulateur électronique (1)selon l'une quelconque des revendications 1 à 14 implanté, pour influer sur la partie de génération d'impulsions de charge, et dans lequel plusieurs bobines (3-1, ..., 3-3) sont prévues pour générer le champ magnétique se modifiant, lesquelles peuvent être pilotées en conséquence sur la base du signal indiquant la qualité de l'impulsion de charge pour une optimisation de l'impulsion de charge.

16. Chargeur (1") selon la revendication 15, dans lequel la fréquence d'inversion de polarité se situe dans une plage de
- X à 10 kHz, dans lequel X > 0 et X > = 0,1 kHz, 0,2 kHz, 0,3 kHz, ..., 4,9 kHz, ..., ou 9,9 kHz.
